# FASCICULE DE BREVET EUROPEEN

(11) **EP 0 855 882 B1**
(45) Date de publication et mention de la délivrance du brevet: **11.02.2004**
(21) Numéro de dépôt: 96925788.0
(22) Date de dépôt: 16.07.1996
(51) Int. Cl.: A61B 17/72, A61B 17/17

(54) **ENSEMBLE POUR L'ENCLOUAGE D'UN CLOU CENTROMEDULLAIRE ASCENDANT DU FEMUR AU VERROUILLAGE MECANIQUE DE SES DEUX EXTREMITES**
FIXATIONSSYSTEM FÜR AUFSTEIGENDEN FEMUR-MARKNAGEL MIT EINER MECHANISCHEN VERRIEGELUNG AN BEIDEN SEITEN
FIXATION SYSTEM FOR ASCENDING CENTROMEDULLARY THIGH BONE PIN WITH MECHANICAL CLAMPING OF ITS TWO ENDS

(43) Date de publication de la demande: 05.08.1998
(73) Titulaire: Centerpulse Orthopedics Ltd., 6340 Baar (CH)
(72) Inventeur: Vichard, Philippe, Prof., 2500 Besancon (FR)
(74) Mandataire: Finsterwald, Martin, Dr.
(86) Numéro de dépôt international: PCT/FR1996/001097
(87) Numéro de publication internationale: WO 1998/002104

(56) Documents cités:
- WO-A-92/01422
- FR-A- 2 668 360
- FR-A- 2 690 330
- US-A- 2 821 979
- US-A- 4 622 959
- US-A- 5 179 915
- US-A- 5 248 313

## Description

Il s'agit d'un clou centromédullaire dit ascendant (c'est-à-dire dont le point d'introduction est situé dans l'articulation du genou), qui se verrouille dans l'épiphyse distale du fémur grâce à un procédé mécanique ; son verrouillage au niveau de la métaphyse proximale du fémur est également mécanique grâce à un artifice décrit plus loin.
Ce clou est destiné à traiter d'une manière générale des fractures situées au niveau du 1/3 distal du fémur, notamment :
- les fractures métaphysaires
- les fractures étagées métaphysaires et diaphysaires
- certaines fractures épiphysaires

L'enclouage centromédullaire, verrouillé ou non, solidarisé au mieux deux fragments d'égale longueur (1). Lorsque le ou un des traits de fracture est proche d'une épiphyse, la meilleure solution mécanique consiste à introduire le clou à proximité de l'épiphyse concernée (2). D'autre part le clou grâce à un long trajet centromédullaire dans le plus gros fragment oriente automatiquement le plus court des deux fragments.

Jusqu'ici, compte-tenu des voies d'abord décrites, les chirurgiens utilisaient, quelle que soit la situation du trait de fracture sur le fémur, l'abord trochantérien. En raison du caractère peu satisfaisant de cet abord le chirurgien renonçait souvent à l'enclouage pour retenir des plaques le plus souvent coudées (3) prenant appui sur l'épiphyse : en effet l'enclouage classique même verrouillé est soumis à des aléas :
1° - Nous avons vu les avantages mécaniques du cathétérisme premier fragment de fémur le plus court
2° - Le clou peut aboutir dans un des condyles ce qui perturbe la réduction et peu conduire au cal vicieux
3° - Des traits de refend épiphysaires peuvent être élargis, des fragments plus ou moins bien identifiés peuvent être mobilisés.
4° - Le verrouillage distal est radioscopique, ce qui est une source de difficulté et d'irradiation du personnel médical et paramédical.

A l'inverse, le recours à la plaque jugé inévitable n'est pas la solution idéale. En effet, il s'agirait d'une intervention à ciel ouvert, extensive, donc source de saignement per-opératoire d'infection, de consolidation difficile.

Un ensemble pour l'enclouage centromédullaire d'un fémur selon le préambule de la revendication 1 est connu du document WO-A-92/01422.

La présente innovation a pour but de permettre un enclouage centromédullaire verrouillé à foyer fermé sans les inconvénients de l'enclouage classique vus plus haut, avec en plus d'autres avantages :
a) le verrouillage mécanique aux deux extrémités du clou
b) l'utilisation d'un nombre de clous limité et d'un matériel ancillaire simple, le chirurgien se servira
   - d'une seule longueur de clou
   - de deux diamètres
   - de clous interchangeables à droite et à gauche

### LÉGENDES

La présente invention est illustrée par les dessins annexés parmi lesquels :

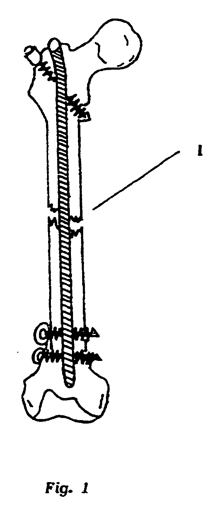

La figure 1 montre un enclouage centromédullaire classique du fémur solidarisant deux fragments diaphysaires d'égale longueur.

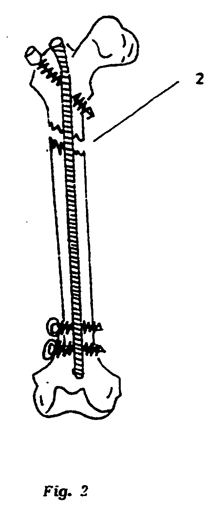

La figure 2 montre un enclouage centromédullaire classique solidarisant deux fragments de longueurs inégales. Comme le trait de fracture est proche du point d'introduction trochantérien du clou, le montage est satisfaisant.

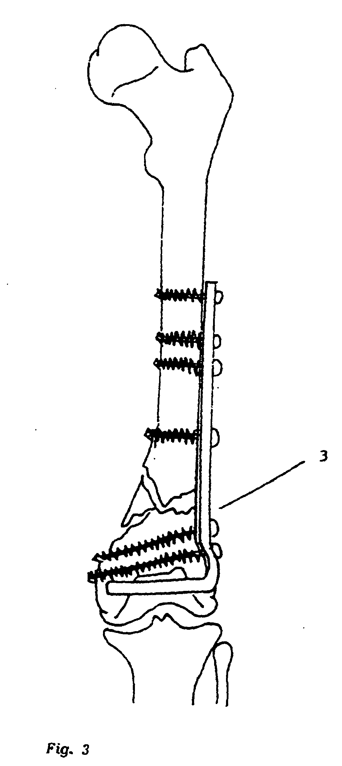

La figure 3 montre une plaque coudée solidarisant les deux principaux fragments lorsque la fracture fémorale siège sur le 1/3 distal du fémur. En effet un clou centromédullaire d'introduction trochantérienne ne serait pas satisfaisant pour différentes raisons précisées dans le texte.
La figure 4 met en évidence un clou centromédullaire ascendant de profil ; ce clou est incurvé dans le plan sagittal.

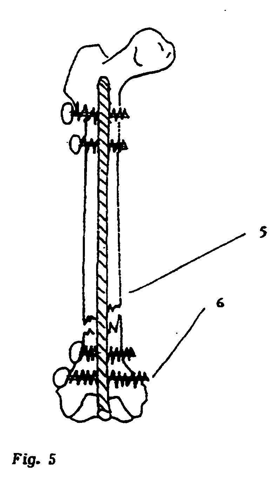

La figure 5 montre le clou ascendant de face.

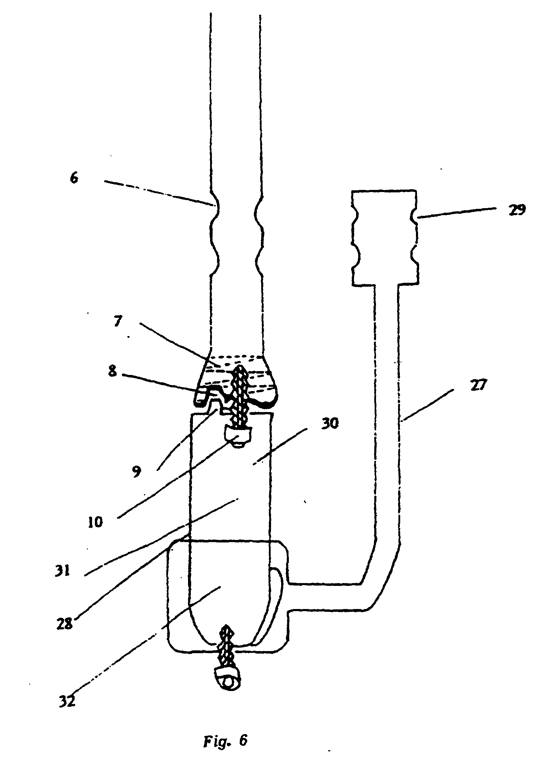

La figure 6 montre l'assemblage clou ascendant - adaptateur - guide universel de verrouillage.

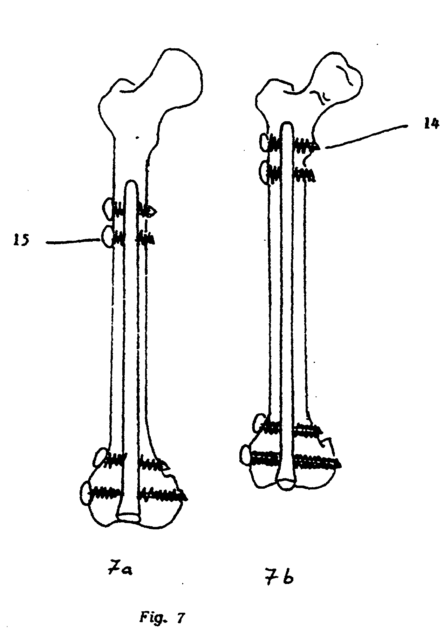

Les figures 7 mettent en évidence la situation variable du verrouillage sous-trochantérien suivant la longueur du fémur traité, dans la mesure où on souhaite utiliser un stock de clou très limité.

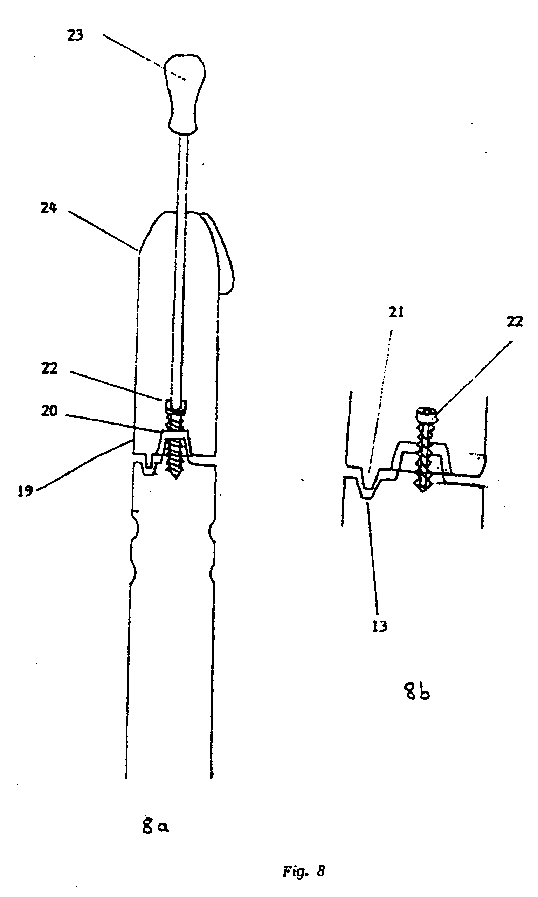

Les figures 8 montrent l'articulation du perforateur guide et du clou.

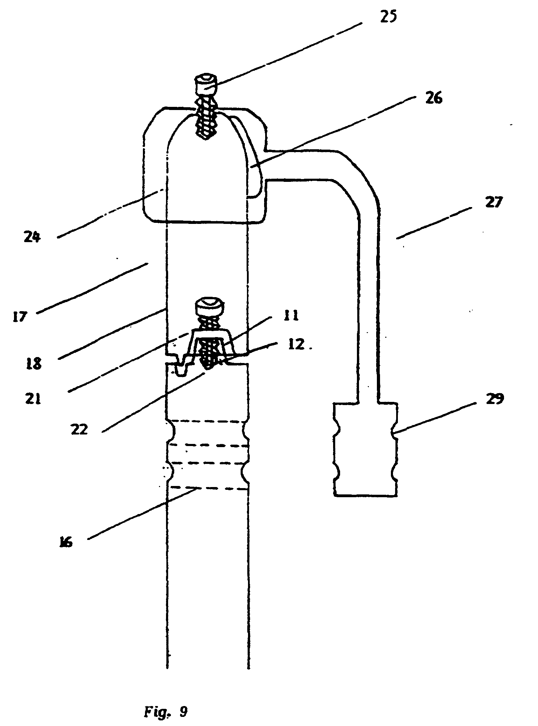

La figure 9 montre l'assemblage clou - perforateur guide et guide universel de verrouillage.

### LE MATÉRIEL

### A. - LE CLOU (4, 5)

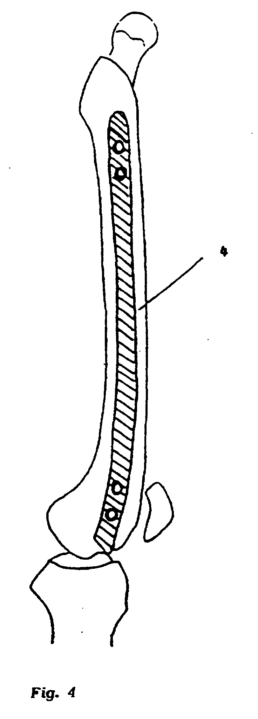

Il s'agit d'un clou (4, 5) plein cylindrique épousant la forme du canal médullaire : il est incurvé dans le plan sagittal (fig 4). Le clou est percé de deux conduits de verrouillage (6) à direction frontale et parallèle très proches de son extrémité inférieure. Nous désignerons par ce terme l'extrémité qui en fin d'intervention est située dans l'articulation du genou.

Au niveau de l'extrémité inférieure du clou un filetage femelle (7) et une petite encoche (8) permettent le vissage d'un ensemble de pièces grossièrement en U dont l'encoche mâle (9) se fixe dans l'encoche femelle du clou. Une vis (10) solidarise ensuite le clou et l'ancillaire.

L'extrémité supérieure (11) du clou (nous désignons par ce terme l'extrémité qui en fin d'intervention est située dans la métaphyse proximale du fémur) est constituée par un cylindre dont la base a un diamètre légèrement inférieur à celui de la portion moyenne du clou. Ce cylindre de dimensions réduites est creux. Cette zone creuse (12) qui occupe ce sommet a une forme cylindrique : elle est filetée pour admettre une vis. A la base du cylindre de dimensions réduites le clou lui-même est porteur d'une encoche femelle (13) destinée à s'articuler avec l'encoche mâle (21) du perforateur guide (voir plus loin).

La longueur du clou est telle qu'il puisse être utilisé au niveau de tous les fémurs, le verrouillage pouvant être réalisé à hauteur du petit trochanter [pour les fémurs les plus courts (14)] à un quelconque niveau du 1/3 proximal du fémur [pour les fémurs plus longs (15)]. Le clou est porteur de deux autres conduits de verrouillage (16) de direction frontale et situés très près du tronc de cône mentionné plus haut.

### B. - MATÉRIEL ANCILLAIRE

### 1°) Le perforateur-guide (17)

De bas en haut il a une forme cylindrique puis effilée. Son plus grand diamètre est égal à celui du clou à son extrémité supérieure (cylindre, destiné à l'articulation avec le perforateur-guide, exclu).
Ce perforateur-guide présente à considérer trois portions :
a) *une portion moyenne* (18) de loin la plus longue, cylindrique parcourue par un cylindre intérieur sur toute sa longueur.
*b) une portion inférieure* (19) également creuse parcourue par un cylindre femelle (20) de dimensions réduites destiné à s'articuler avec celui mâle de l'extrémité supérieure du clou. Une cannelure mâle (21) s'articule avec l'encoche femelle (13) de l'extrémité supérieure du clou.
   Une vis (22) est mise en place grâce à un long tournevis (23). La tête de la vis a un diamètre supérieur à celui du sommet du petit cylindre femelle.
*c) une portion supérieure* (24) : les différents diamètres du perforateur-guide se réduisent progressivement ce qui donne grossièrement à l'ensemble clou - perforateur-guide la forme d'un obus.
   Cette portion supérieure est également creuse, et la cavité interne constitue un cylindre de même diamètre que celui de la portion moyenne.
   Mais la paroi de ce cylindre est parcourue par un filetage ; une vis (25) introduite grâce à ce filetage obture le canal central du perforateur-guide ou le solidarise avec le guide universel de verrouillage (dont il sera fait mention plus loin).
   La surface externe de cette portion supérieure est parcourue par une crête acérée longitudinale (26) qui atteint l'extrémité supérieure du perforateur et qui s'articulera avec une crête femelle du guide universel de verrouillage (voir ci-après).

### 2° Le guide universel de verrouillage (27)

Il est dit universel car il sert au verrouillage inférieur comme au verrouillage supérieur du clou grâce à l'utilisation d'un adaptateur (28) qui remplace pour le verrouillage inférieur, le perforateur-guide, utilisé pour le verrouillage supérieur.

Le guide universel de verrouillage a une disposition en équerre. C'est son articulation avec le perforateur-guide ou l'adaptateur qui confère à l'ensemble une forme globale en U. Allongé, en équerre il a une section quadrangulaire.

Il est perforé de deux conduits (29) cylindriques parallèles rapprochés à direction frontale.

La position de ces deux conduits cylindriques est calculée pour qu'une mèche percutanée atteigne sans difficulté les orifices de verrouillage des extrémités inférieure ou supérieure du clou. De la même manière les cannelures, les crêtes (26) (mâles ou femelles) du clou et du perforateur-guide (17), de l'adaptateur, du canon de verrouillage sont disposées pour permettre cette visée mécanique.

### 3° L'adaptateur (28)

Il a une forme extérieure analogue à celle du perforateur-guide. Son plus grand diamètre est destiné à s'adapter à l'extrémité inférieure du clou grâce à la cavité cylindrique femelle filetée de ce dernier qui admet une vis. II présente à considérer trois portions : une base, un corps et un sommet.

*La base de l'adaptateur* (30) (nous désignons ainsi son extrémité assujettie au clou dans l'articulation du genou) encochée est porteuse d'une cannelure mâle (9), adaptée à la cannelure femelle (8) dont est porteuse l'extrémité inférieure du clou. Une vis centromédullaire solidarise clou et adaptateur, les cannelures étant judicieusement articulées. Par ailleurs, l'intérieur de cette base est parcouru par une cavité cylindrique interrompue à son extrémité articulaire, le clou, par un diaphragme traversé par la vis adaptée au filetage du clou.

*La portion moyenne (31) de l'adaptateur* est analogue à celle du perforateur-guide;

*Le sommet (32) de l'adaptateur* est en tous points analogue à celui du perforateur-guide de manière à s'articuler avec le guide universel de verrouillage.

### LA TECHNIQUE DE L'ENCLOUAGE

Le malade est en décubitus dorsal sur table normale, une barre transversale surélevant le fragment proximal du fémur. Pour les fractures très distales, il est possible d'utiliser un garrot pneumatique pour l'hémostase préventive.

Le champ opératoire comprend tout le membre inférieur qui peut être manipulé par un aide. Le champ opératoire inclut la région de la hanche, un amplificateur de brillance visualise la réduction de la fracture, surveille la progression du clou mais ne joue aucun rôle dans le verrouillage.

La traction longitudinale réalisée par un aide peut être facilitée par une broche transtibiale.
**1°) Repérage de l'orifice d'entrée du clou dans le genou**
   Le genou est fléchi par l'aide. Une courte incision para-rotulienne interne permet de repérer avec l'index l'orifice d'entrée qui se situe dans l'échancrure intercondylienne juste en arrière du cartilage et en avant des insertions des ligaments croisés. Une pointe carrée introduite au ras de la paume de la main, au contact de la pulpe de l'index ouvre le canal médullaire.
   L'orifice est progressivement élargi grâce à des pointes carrées de taille croissante. Le perforateur-guide est fixé sur le clou et l'ensemble est poussé dans le canal médullaire, sans alésage préalable ; toutefois dans certaines circonstances particulières (canal médullaire modifié par des lésions ou des interventions antérieures) un alésage préalable à l'enclouage peut être réalisé.
**2° L'ensemble clou - perforateur-guide** est poussé dans le canal médullaire. Il atteint le "sablier" (zone rétrécie en diabolo du canal médullaire au 1/3 moyen). Et dès qu'il l'a franchi (vérification par l'amplificateur de brillance) le foyer de fracture se trouve réduit dans les plans frontal et sagittal. En effet, le point d'entrée et deux points au moins du canal médullaire se trouvent alignés.

Toutefois l'opérateur doit rester vigilant vis-à-vis de la rotation axiale du fémur distal, du télescopage possible des principaux fragments. Enfin, une manipulation trop énergique de l'épiphyse avant verrouillage inférieur pourrait entraîner un déplacement de cette épiphyse dans les plans frontal et sagittal grâce à une bascule de cette épiphyse autour de l'orifice d'entrée du clou et compte-tenu d'une fracture éventuellement très comminutive.
L'ensemble clou - perforateur-guide plus long que toutes les variétés de fémur progresse et perfore le sommet du trochanter. C'est ici que se situe le verrouillage inférieur du fémur à l'aide de l'ensemble adaptateur - guide universel de verrouillage.

C'est une bonne pratique si on veut éviter, comme souligné plus haut, la bascule de l'épiphyse autour de l'orifice d'entrée du clou. Cependant le verrouillage inférieur peut être réalisé plus tard.
Ce verrouillage inférieur comporte les gestes suivants :
a) vissage de la base de l'adaptateur sur le clou grâce à un long tournevis qui parcourt le canal interne de cet adaptateur pour le visser au clou
b) mise en place et vissage du guide universel de verrouillage
c) verrouillage proprement dit c'est-à-dire :
   - mise en place de mèche dans les conduits de verrouillage du guide universel permettant :
      . le forage du fémur (deux corticales). Ce forage a été précédé d'une incision cutanée et d'une préparation du forage cortical grâce à une pointe carrée adaptée ;
   - mensuration de la longueur de la vis ;
   - mise en place des deux vis de verrouillage

Après la traversée du sommet du trochanter, le perforateur-guide (d'où son nom) poussé par le clou refoule les muscles et fait saillir la peau d'autant que l'aide aura porté la hanche en adduction. La peau de la hanche est incisée ; on voit le perforateur-guide.
Il suffit alors d'adapter le guide universel de verrouillage sur le perforateur et de procéder au verrouillage supérieur dans des conditions identiques à celles du verrouillage inférieur.

Le guide universel de verrouillage, le perforateur-guide sont enlevés grâce à des manoeuvres inverses de celles qui ont présidé à leur mise en place.
Points séparés sur les téguments de la hanche. Fermeture du plan synovialo-aponévrotique et du plan cutané du genou.

### SOINS POST-OPÉRATOIRES

Le genou et la hanche peuvent être immédiatement mobilisés.

### LEGENDES DES SCHEMAS

- 1.: Enclouage centromédullaire classique solidarisant deux fragments diaphysaires d'égale longueur.
- 2.: Enclouage centromédullaire classique avec point d'introduction trochantérien du clou. Le montage est mécaniquement satisfaisant car le fragment proximal est plus court que le distal.
- 3.: Plaque coudée solidarisant les deux principaux fragments lorsque la fracture fémorale siège sur le 1/3 distal de l'os.
- 4.: Clou centromédullaire ascendant incurvé dans le plan sagittal.
- 5.: Clou ascendant de face.
- 6.: Les deux conduits de verrouillage de l'extrémité inférieure du clou centromédullaire ascendant.
- 7.: Filetage situé au niveau de l'extrémité inférieure creuse du clou ascendant et permettant la solidarisation de l'adaptateur et du clou.
- 8.: Encoche femelle de l'extrémité inférieure du clou.
- 9.: Encoche mâle de l'adaptateur.
- 10.: Vis solidarisant l'adaptateur et l'extrémité inférieure du clou.
- 11.: Extrémité supérieure du clou et son cylindre creux.
- 12.: Filetage occupant les parois du cylindre creux. A l'intérieur le cylindre est creux. La cavité est cylindrique. Les parois sont revêtues d'un filetage.
- 13.: Encoche femelle du clou située à la jonction tronc de cône portion moyenne du clou.
- 14.: Pour les fémurs courts, le verrouillage de l'extrémité supérieure du clou est proche du massif trochantérien.
- 15.: Pour les fémurs longs, le verrouillage de l'extrémité supérieure du clou est situé beaucoup plus loin du massif trochantérien.
- 16.: Conduits de verrouillage de l'extrémité supérieure du clou.
- 17.: Le perforateur-guide.
- 18.: Portion moyenne du perforateur-guide.
- 19.: Portion inférieure du perforateur-guide.
- 20.: Cylindre femelle s'articulant avec le cylindre mâle du clou.
- 21.: Cannelure mâle s'articulant avec la cannelure femelle de l'extrémité supérieure du clou.
- 22.: Vis solidarisant les deux cylindres mâle et femelle.
- 23.: Tournevis à longue tige permettant la mise en place de la vis solidarisant clou et perforateur-guide.
- 24.: Portion supérieure du perforateur-guide;
- 25.: Vis, qui est introduite dans le filetage tapissant le cylindre creux, occupant l'extrémité supérieure du perforateur, lequel prolonge le cylindre creux de la portion moyenne.
- 26.: Crête acérée longitudinale, qui s'articulera avec la crête femelle du guide universel de verrouillage.
- 27.: Le guide universel de verrouillage.
- 28.: L'adaptateur.
- 29.: Les conduits de verrouillage du guide universel.
- 30.: La base de l'adaptateur.
- 31.: Portion moyenne de l'adaptateur.
- 32.: Sommet de l'adaptateur.

## Revendications

1. Ensemble pour l'enclouage centromédullaire d'un fémur comportant un clou (4, 5) plein, cylindrique pour épouser la forme du canal médullaire et incurvé dans le plan sagittal, ledit clou étant destiné à être introduit par l'échancrure intercondylienne et présentant près de son extrémité inférieure et de son extrémité supérieure deux conduits parallèles (6, 16) perpendiculaires au plan sagittal destinés à recevoir des vis de verrouillage, et un guide de verrouillage en forme d'équerre, ledit guide de verrouillage (27) étant perforé par deux conduits cylindriques parallèles (29) destinés à permettre la mise en place des vis de verrouillage dans deux conduits parallèles (6, 16) dudit clou (4, 5),
**caractérisé par le fait que** ledit clou (4, 5) présente à son extrémité inférieure un premier conduit fileté (7) et une première encoche femelle (8) , et à son extrémité supérieure un deuxième conduit cylindrique fileté (12) et une deuxième encoche femelle (13), et
**par le fait que** ledit ensemble comporte en outre:
- un perforateur-guide (17) susceptible d'être vissé de manière amovible à l'extrémité supérieure dudit clou (4, 5) , ledit perforateur-guide présentant une portion moyenne (18) de forme cylindrique, dont le plus grand diamètre est égal à celui de l'extrémité supérieure dudit clou (4, 5) et qui comporte un évidemment cylindrique sur toute sa longueur, une portion inférieure (19) comportant une cannelure mâle (21) destinée à s'articuler sur la deuxième encoche femelle (13) dudit clou (4, 5), et une portion supérieure (24) effilée, mais creusée en son sein d'un cylindre fileté femelle, et qui présente une crête acérée longitudinale (26) ;
- un adaptateur (28) susceptible d'être vissé de manière amovible à l'extrémité inférieure dudit clou (4, 5), ledit adaptateur présentant une base (30) comportant une cannelure mâle s'adaptant sur la première encoche femelle (8) dudit clou (4, 5), une portion moyenne (31) conforme à la portion moyenne (18) dudit perforateur-guide (17), et un sommet (32) conforme à la portion supérieure (24) dudit perforateur-guide (17) ; et **par le fait que**
- le guide de verrouillage (27) est susceptible d'être fixé sur ledit perforateur- guide (17) ou sur ledit adaptateur (28) au moyen d'une vis (25) coopérant avec ledit cylindre fileté femelle.

## Patentansprüche

1. Einheit für die Marknagelung eines Femur, umfassend einen vollen Nagel (4, 5), der zylindrisch ist, um sich an die Form des Markkanals anzupassen, und in der Sagittalebene gekrümmt ist, wobei dieser Nagel dazu bestimmt ist, über die interkondyläre Aufnahme eingeführt zu werden, und in Nähe seines unteren Endes und seines oberen Endes zwei parallele, zur Sagittalebene senkrechte Kanäle (6, 16) aufweist, die dazu bestimmt sind, Verriegelungsschrauben aufzunehmen, und eine winkelförmige Verriegelungsführung, wobei diese Verriegelungsführung (27) zwei parallele zylindrische Kanäle (29) aufweist, die dazu bestimmt sind, das Einsetzen der Verriegelungsschrauben in zwei parallele Kanäle (6, 16) des Nagels (4, 5) zu gestatten,
**dadurch gekennzeichnet, daß** der Nagel (4, 5) an seinem unteren Ende einen ersten mit Gewinde versehenen Kanal (7) und eine erste aufnehmende Vertiefung (8) und an seinem oberen Ende einen zweiten zylindrischen, mit Gewinde versehenen Kanal (12) und eine zweite aufnehmende Vertiefung (13) aufweist, und
dadurch, daß diesen Einheit außerdem aufweist:
- einen Führungsperforator (17), der am oberen Ende des Nagels (4, 5) lösbar angeschraubt werden kann, wobei dieser Führungsperforator einen mittleren Teil (18) zylindrischer Form, dessen größter Durchmesser gleich dem des oberen Endes des Nagels (4, 5) ist und der auf seiner ganzen Länge eine zylindrische Aussparung aufweist, einen unteren Teil (19) mit einer einsteckbaren Erhebung (21), die dazu bestimmt ist, sich an der zweiten aufnehmenden Vertiefung (13) des Nagels (4, 5) anzulenken, und einen oberen Teil (24) aufweist, der sich verjüngt, in dem jedoch ein mit Gewinde versehener aufnehmender Zylinder ausgebohrt ist und der einen scharfen Längsgrat (26) aufweist;
- einen Adapter (28), der am unteren Ende des Nagels (4, 5) lösbar angeschraubt werden kann, wobei dieser Adapter eine Basis (30), die eine einsteckbare Erhebung aufweist, die sich in die erste aufnehmende Vertiefung (8) des Nagels (4, 5) einpaßt, einen dem mittleren Teil (18) des Führungsperforators (17) entsprechenden mittleren Teil (31) und einen dem oberen Teil (24) des Führungsperforators (17) entsprechenden Scheitel (32) besitzt; und dadurch, daß
- die Verriegelungsführung (27) an dem Führungsperforator (17) oder an dem Adapter (28) mit Hilfe einer Schraube (25) befestigt werden kann, die mit dem mit Gewinde versehenen aufnehmenden Zylinder zusammenwirkt.

## Claims

1. Assembly for intramedullary nailing of a femur, comprising a solid cylindrical nail (4, 5) matching the shape of the medullary canal and inwardly curved in the sagittal plane, said nail being intended to be introduced via the intercondylar notch and having, near its lower end and near its upper end, two parallel conduits (6, 16) which are perpendicular to the sagittal plane and are intended to receive locking screws, and a locking guide in the form of an angled bracket, said locking guide (27) being holed with two parallel cylindrical conduits (29) which are intended to permit positioning of the locking screws in two parallel conduits (6, 16) of said nail (4, 5),
**characterized in that** said nail (4, 5) has, at its lower end, a first threaded conduit (7) and a first female notch (8) and, at its upper end, a second threaded cylindrical conduit (12) and a second female notch (13), and
**in that** said assembly additionally comprises:
- a guide drill (17) which can be screwed detachably to the upper end of said nail (4, 5), said guide drill having a middle portion (18) of cylindrical shape, the greatest diameter of which is equal to that of the upper end of said nail (4, 5) and which comprises a cylindrical recess along its entire length, a lower portion (19) comprising a male rib (21), intended to articulate with the second female notch (13) of said nail (4, 5), and an upper portion (24) which is narrowed, but hollowed out on its inside with a female threaded cylinder, and which has a sharp longitudinal crest (26) ;
- an adapter (28) which can be screwed detachably to the lower end of said nail (4, 5), said adapter having a base (30) with a male rib fitting on the first female notch (8) of said nail (4, 5), a middle portion (31) corresponding to the middle portion (18) of said guide drill (17), and a summit (32) corresponding to the upper portion (24) of said guide drill (17); and **in that**
- the locking guide (27) can be fixed on said guide drill (17) or on said adapter (28) by means of a screw (25) cooperating with said female threaded cylinder.
